(19) Europäisches Patentamt
European Patent Office
Office européen des brevets

(11) Publication number: 0 226 320 B1

(12) EUROPEAN PATENT SPECIFICATION

(45) Date of publication of the patent specification: 03.10.90

(51) Int. Cl.[5]: C07D 495/04, A61K 31/55 // (C07D495/04, 337:00, 333:00)

(21) Application number: 86308622.9

(22) Date of filing: 05.11.86

(54) Imidazolyl thieno-benzothiepin derivatives and processes for the preparation thereof.

(30) Priority: 29.11.85 CS 8664/85

(43) Date of publication of application: 24.06.87 Bulletin 87/26

(45) Publication of the grant of the patent: 03.10.90 Bulletin 90/40

(84) Designated Contracting States: AT BE CH DE FR GB IT LI LU NL SE

(56) References cited:
GB-A-1 081 360

CHEMICAL ABSTRACTS, vol. 67, no. 19, 06 Nov 1967, Columbus, OH (US); M.RAJSNER et al.: "Neurotropic and psychotropic substances. XV. 4,9-dihydrothieno[2,3-b]benzo[e]thiepin derivatives", pp. 8536-8537, no. 90702r

(73) Proprietor: SPOFA Spojené Podniky Pro Zdravotnickou Vyrobu, Husinecká 11 a, Prag 3(CS)

(72) Inventor: Leiner, Jan, No 35 Lidovych milci, Praha 2(CS)
Inventor: Holá, Vladislava, No 17 Bratri Capku, Praha 10(CS)
Inventor: Rajsner, Miroslav, No 10 Pod Krariovem, Praha 2(CS)
Inventor: Matunova, Iva, No 1741 Kvetnového vitezstvi, Praha 4(CS)

(74) Representative: Arthur, John William et al, FITZPATRICKS 4 West Regent Street, Glasgow G2 1RS Scotland(GB)

EP 0 226 320 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

ACTORUM AG

## Description

The present invention relates to imidazolyl thieno-benzothiepin derivatives of the general formula I

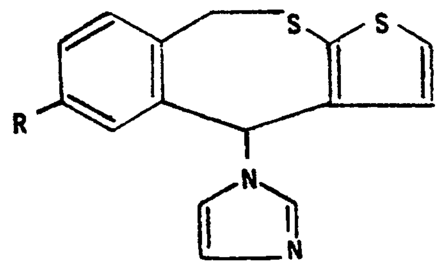

(I)

in which R is a hydrogen or a chlorine atom, addition salts thereof with pharmaceutically acceptable inorgantic and organic acids as well as to processes for their preparation.

Recent promotion in the use of antibiotics, cytostatics, immuno-suppressive agents and hormonal therapy underlines the clinical importance of mycotic infections, which not seldom result in generalization of the disease and death of the patient. Pathogenic yeasts and moulds appear as the most frequent aetiological factors that induce dermal and related infections. Also gynaecological practice reports rising frequency of vaginal mycosis cases. Therapy of epidermal and gynaecological mycoses has become a difficult problem, ie, due to a wide variety of originators and rapidly developing resistance. Despite the fact that numerous antimycotic agents of diverse chemical structures and modes of action were tested and partly introduced into practice, there is encountered steady shortage of potent, reliable and safe remedies both for the prevention and curative treatment of mycotic infections.

It has recently been found that the subject compounds of the invention, imidazolyl thieno-benzothiepins of formula I, in which R has the above-defined meaning, and their acid addition salts possess remarkable antimycotic properties. Thus, an in vitro comparison was made between minimum inhibitive concentrations (MIC) of these compounds and those of commercial antimycotic agents clotrimazole, miconazole and ketoconazole (nonproprietory generic names). The MIC values were determined by the standard dilution method in the prescribed liquid nutrient medium primarily with the use of the following typical yeast and mould species currently employed in preliminary antimycotic screening: Saccharomyces pastorianus (SP), Candida albicans (CA), Trichophyton mentagrophytes (TM) and Aspergillus niger (AN). The test inoculum size was $10^6$ $CFU.ml^{-1}$ (CFU is a standard abbreviation for microbial cell counts) in the mould strains (AN, TM) and $10^4$ $CFU.ml^{-1}$ in the yeast and related species (CA and SP).

The following Table I summarizes experimental results of the preliminary in vitro screening of the two compounds of the invention, ie, compound Ia (R = H, VÚFB 15018) and compound Ib (R = Cl, VÚFB 16208), in comparison with the aforementioned known antimycotic agents in clinical use. The MIC values are given in milligrams of the respective compound per litre of the medium, and the above explained abbreviations for the used test microorganisms are employed.

TABLE 1

Antimycotic activity (MIC, mg per l) to yeast and mould species

| Compound | SP | CA | TM | AN |
|---|---|---|---|---|
| Clotrimazole | 3.1 | 0.7 | 0.03 | 3.1 |
| Miconazole | 6.2 | 0.7 | 0.03 | 1.5 |
| Ketoconazole | 50 | 0.03 | 0.7 | 12.5 |
| Compound Ia | 25 | 25 | 1.5 | 0.7 |
| Compound Ib | 12.5 | 3.1 | 0.03 | 0.03 |

The sensititivy of a larger number of clinical strain isolates of pathogenic moulds (16 in total) as well as yeasts and related microorganisms (21 in total) to the subject compounds of formula I was determined using the dilution method on Sabouraud agar. Thus, the experimental MIC values for mould strains and compound Ia were in the range of from 0.7 to 100 mg/l, for compound Ib from 0.01 to 1.5 mg/l, and for clotrimazole, which is the most potent antimycotic compound out of those listed above as taken for comparison, the MIC values similarly ranged from 0.01 to 1.5 mg/l. Addition of serum or blood to the test plate agar enhanced twice the effect of compound Ia, did not affect the potency of compound Ib, and diminished the activity of clotrimazole to about a third against the protein-free agar culture value.

The in vivo evaluation of the antimycotic activity was made using a model of vaginal candidosis induced experimentally by Candida albicans species in mice. The substances under test were administered over a period of 10 days intravaginally in the form of homogenized suspensions of appropriately chosen concentrations in 0.5% aqueous hydroxyethylcellulose solution. The results were evaluated both immediately on administration thereof and after the elapse of one and two weeks. Compound Ia at a concentration of 7.5% elicited complete inhibition of the test culture growth both in therapy starting 24 hours prior to and simultaneously with the experimental infection, ie, the compound also had a significant prophylactic preventive effect. In case of delayed therapy starting 24 hours after the infection, the experimental results were somewhat less favourable in all of the substances involved.

Among the subject compounds of formula I, the aforementioned compound Ib (clopinazole, preliminary nonproprietory name) is the substance of choice. When compared with clotrimazole and miconazole, which are the heretofore most potent antimycotic agents derived from imidazole, this compound Ib showed similar activity against typical inducers of dermal mycoses at markedly increased activity towards Aspergillus genus strains; to further advantage its potency is not diminished by the presence of biological protein substrates such as blood constituents. Experimental treatment of vaginal Candida albicans infection in mice confirmed the beneficial therapeutical effect of the compound also on topical application. Minimum inhibitive concentration (MIC) values of compound Ib, in the dilution test on Sabouraud agar, for about forty pathogenic yeast and mould strains ranged from 0.01 to 25 mg/l. This compound has only slight toxic properties: its $LD_{50}$ in female and male mice was respectively 1125 and 1570 mg/kg p o. The standard tests for mutagenity, cancerogenity, teratogenity (foetal damage), dermal and vaginal irritation as well as chronic (one-month) toxicity in rat and dog on intravaginal administration were all negative. The promising pharmacological results concerning compound Ib allow one to expect favourable, beneficial effects in therapeutical practice.

The reported in vitro test results allow one to assume that the novel imidazolyl thieno-benzothiepin derivatives of formula I, especially the aforementioned compound Ib, can find use for the treatment of epidermal and epithelial (eg, vaginal) mycoses in mammals. For the purpose of therapeutical application, the active compounds can advantageously be formulated, using appropriate common formulating auxiliaries, into convenient topical and oral dosage forms such as, eg, solutions, suspensions, gels, creams, ointments, powders, tablets, capsules, sprays and other typical means of administration. These can contain the active ingredient preferably in a quantity substantially in the range of from 1 to 10% by weight.

The subject compounds of the present invention, imidazolyl thieno-benzothiepin derivatives of the general formula I

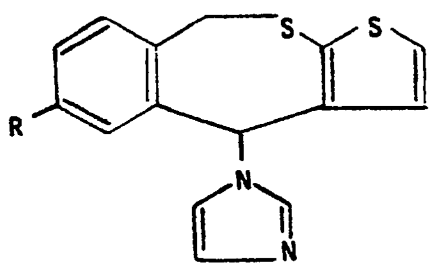

(I)

in which R has the afore defined meaning, and their acid addition salts can be prepared according to the invention by several related methods which comprise reacting the respective thieno-benzothiepin-4-ol compound of the general formula II

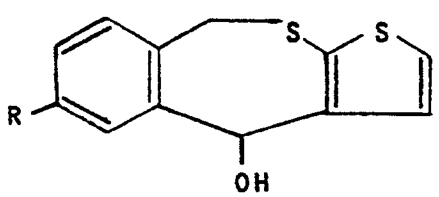

(II)

in which R has the same meaning as in formula I, or its alkane- or arenesulphonate ester with imidazole or a reactive derivative thereof, preferably in the medium of an inert organic solvent, and optionally, if required with subsequent neutralization of the resultant base with a pharmaceutically acceptable inorganic or organic acid.

Starting 4,9-dihydrothieno (2,3-c)-2-benzothiepin-4-ol is a known substance (M Rajšner et al, Collection Czechoslovak. Chem. Commun. 32, 2864, 1967). The alternatively required 6-chloro derivative of this compound is new, available by reduction of 6-chlorothieno (2,3-c)-2-benzothiepin-4(9H)-one (idem, 1.c.) with hydride reducing agents, eg sodium borohydride in methanol or ethanol.

The reaction of said thieno-benzothiepin-4-ol compounds with free imidazole can be effected by heating a mixture of these reactants for a period of 2 to 5 hours to a temperature of 140 to 170°C, or by refluxing a solution of the mixture in a higher-boiling solvent, eg commercial xylene, for 3 to 5 hours at the boiling temperature. After optional removing of the solvent, the product is isolated and purified by crystallization, eg from toluene.

Alternatively, the starting thieno-benzothiepin-4-ol can first be reacted with an alkane- or arenesulphonyl-chloride, eg p-toluene-sulphonylchloride, in the presence of an alkaline agent capable to bind the formed hydrogen chloride, preferably anhydrous pyridine, to give a reactive alkane- or arenesulphonyl ester, and subsequently reaction of this intermediate in situ with free imidazole is effected. The product is isolated by diluting the reaction mixture with water, extraction into an appropriate solvent, eg chloroform, removal of the solvent and crystallization of the residue.

The starting thieno-benzothiepin-4-ol can advantageously be reacted with 1,1'-carbonyldiimidazole in an anhydrous aromatic hydrocarbon, eg benzene or toluene, a chlorinated alkane, eg chloroform, dichloromethane or 1,2-dichloroethane, an aliphatic or cyclic ether, eg tetrahydrofuran, or an aliphatic nitrile, eg acetonitrile, at a temperature substantially in the range of from 10 to 60°C.

The product is isolated either by dispersing the reaction mixture in water and subsequently distilling off the solvent from the separated organic portion (solution of the crude product) or vice versa by removal of the solvent from the whole reaction mixture, and subsequently dispersing the residue in a mixture of water and an appropriate organic solvent, eg ether or toluene, during which operation the product may crystallize.

The starting thieno-benzothiepin-4-ol compound can also be advantageously reacted with 1,1'-thionyldiimidazole, which is preferably formed immediately in the reaction mixture from imidazole and thionylchloride. The latter agent is dropped into a stirred solution or suspension of imidazole in an anhydrous organic solvent such as an aromatic hydrocarbon, a chlorinated alkane or an aliphatic nitrile (convenient examples vide above) at a temperature substantially in the range of from 5 to 20°C. The so-obtained 1,1'-thionyldiimidazole solution containing in suspension the simultaneously formed imidazole hydrochloride is admixed with said starting thieno-benzothiepin-4-ol of formula II and the reaction is allowed to proceed at a temperature substantially from 10 to 25°C. The product is isolated similarly as described in the preceding paragraph.

Still alternatively, the starting thieno-benzothiepin-4-ol can also be reacted with tris(1-imidazolyl) phosphine, prepared by the reaction of imidazole with phosphorus trichloride, or with penta(1-imidazolyl) phosphoran, formed similarly from imidazole and phosphorus pentachloride, or finally with 1,1',1''-phosphonyltriimidazole, obtained in situ by reacting imidazole with phosphonylchloride. When employing any of these three last-mentioned preparative routes which involve organophosphorus agents, the required reactive derivatives of imidazole are conveniently obtained by dropping a chloroform solution of imidazole into a solution of the corresponding phosphorus chloride reagent in the same solvent under stirring and cooling the reaction mixture to a temperature of -5 to 10°C. Upon admixture of a chloroform solution of the starting thieno-benzothiepin-4-ol the solvent is removed under reduced pressure and replaced by an inert solvent with a higher boiling temperature, preferably dimethylformamide, and the reaction is completed by heating for 1 to 3 hours at 120 to 140°C. The product is isolated on cooling by pouring the reaction mixture into water, extraction with an organic solvent, eg chloroform, and evaporation of the volatiles, preferably under reduced pressure.

The resulting crude products are purified either by crystallization from appropriate solvents, eg methanol, ethanol, toluene or a methanol-ether mixture, or by converting by neutralization into addition salts with pharmaceutically acceptable inorganic or organic acids, preferably nitric acid. The bases are liberated from their addition salts by conventional procedures, eg by alkalization of their suspension with alkali metal hydroxides or carbonates, and isolated by extraction into a suitable solvent, eg chloroform, and subsequent evaporation.

The invention will now be further described by way of the following illustrated examples.

Example 1

4-(1-Imidazolyl)-4,9-dihydrothieno(2,3-c)-2-benzothiepin

Method A

4,9-Dihydrothieno(2,3-c)-2-benzothiepin-4-ol (23.4 g) is dissolved by gentle warming to about 30°C in 750 ml of anhydrous benzene. The obtained solution is cooled to 20°C, 1,1'-carbonyldiimidazole (32.4 g) is added in one portion under stirring, and the mixture is stirred for 10 minutes at the same temperature. During the next 15 minutes the temperature is raised to 60°C, and thereafter the reaction mixture is allowed to cool slowly to 20°C and stirred at this temperature over a period of 6 hours. Benzene is distilled off under reduced pressure and the residue is mixed with a water (300 ml) - ether (200 ml) system. Crystals of the product (24.6 g, 86.6% of theory) are separated and dried. The substance has a m.p. of 175 to 177°C. Recrystallization from benzene or methanol yields analytically pure compound; its m.p. is unchanged.

Method B

To a solution of imidazole (27.2 g) in anhydrous acetonitrile (300 ml) is dropped under stirring and ice water cooling to 10°C over a period of about 20 minutes 7.3 ml of thionylchloride. The mixture is stirred for 10 minutes at 10°C and treated portionwise during further 5 minutes under continued stirring and cooling with 4,9-dihydrothieno(2,3-c)-2-benzothiepin-4-ol (23.4 g), wherein the temperature of the mixture is maintained between 10 and 14°C by external ice and water cooling. The reaction mixture is stirred at this temperature for a futher 4 hours. Thereafter, the cooling bath is removed and the mixture is allowed to stand for about 16 hours, concentrated under reduced pressure to a volume of about 50 ml and diluted with 100 ml of water. The crystalline precipitate is taken into chloroform (100 ml), the chloroform solution is washed with water (50 ml), dried over anhydrous sodium sulphate and the solvent is evaporated under reduced pressure. The solid residue (26.8 g, m.p. 162 to 170°C) is crystallized from methanol to give the pure product (15.1 g, 53.2% of theory) with a m.p. of 174.5 to 176.5°C. Acidification of the methanolic solution with nitric acid and admixture with ether yields crystalline nitrate salt, m.p. 143 to 146°C (from methanol-ether).

Method C

A mixture of 4,9-dihydrothieno(2,3-c)-2-benzothiepin-4-ol (2.3 g) and imidazole (1.4 g) is heated for 2 hours at 160°C (bath temperature). The obtained dark melt is cooled and mixed with toluene (5 ml), and the so-formed crystals are separated and washed successively with this solvent (50 ml) and water to give the title product (1.8 g, 63.4% of theory) with a m.p. of 172 to 175°C. The material can be further purified if required by crystallization from methanol.

Method D

To a solution of phosphorus trichloride (1.4 g) in dry chloroform (30 ml) is added under stirring and cooling to a temperature of -5 to 0°C over a period of 1 hour a solution of imidazole (1.4 g) in the same solvent (20 ml). The mixture is allowed to warm up spontaneously to about 20°C and stirring is continued at this temperature for 1 hour. Thereafter a solution of 4,9-dihydrothieno (2,3-c)-2-benzothiepin-4-ol (2.3 g) in dry chloroform (20 ml) is added with continued stirring, whereupon the mixture is stirred for 20 minutes and the solvent is removed under reduced pressure. The residue is refluxed for 2 hours at 130°C with dimethylformamide (20 ml). After cooling, the mixture is diluted with water (50 ml), the product is shaken into chloroform (100 ml), the organic extract is washed with water, dried over anhydrous sodium sulphate, the solvent is evaporated under reduced pressure to a final volume of about 5 ml and the oily residue is allowed to crystallize to give the first-crop product (1.2 g) melting at 174 to 176°C. Concentration of the mother liquor yields further 0.7 g of the second-crop material.

Method E

To a solution of phosphorus pentachloride (2.1 g) in dry chloroform (40 ml) is dropped over a period of about 30 minutes under stirring and cooling to a temperature of 0 to 5°C a solution of imidazole (6.8 g) in 20 ml of dry chloroform. The reaction mixture is allowed to warm to about 20°C, stirring is continued at this temperature for 1 hour and a solution of 4,9-dihydrothieno(2,3-c)-2-benzothiepin-4-ol (2.3 g) in the same solvent (20 ml) is added. The solvent is distilled off under reduced pressure, the residue is mixed with anhydrous dimethylformamide (20 ml), and the mixture is refluxed for 2 hours at 130°C (bath) and diluted with 150 ml of water. The product is shaken into chloroform (100 ml), the organic portion is washed with water, dried over anhydrous sodium sulphate and evaporated under reduced pressure to a small volume (about 5 ml). The crystalline product (1.9 g) melts at 173 to 176°C.

Method F

A solution of phosphonylchloride (1.5 g) in dry chloroform (30 ml) is treated dropwise over a period of 1 hour under stirring and cooling to a temperature of 0 to 5°C with a solution of imidazole (4.1 g) in the same solvent (25 ml). The temperature is then allowed to rise to about 20°C, a solution of 4,9-dihydrothieno-(2,3-c)-2-benzothiepin-4-ol (2.3 g) in dry chloroform (20 ml) is added with stirring, and after a further period of about 20 minutes of continued stirring, the solvent is removed under reduced pressure. The residue is admixed with anhydrous dimethylformamide (20 ml) and the mixture is heated for 2 hours at a bath temperature of 130°C. Thereafter, 150 ml of water is added, the product is shaken into chloroform, the organic portion is washed with water, dried over anhydrous sodium sulphate and the solvent is distilled off under reduced pressure. The residue is mixed with 5 ml of toluene, and the formed crystals are separated and washed with a small volume of the same solvent to yield 2.0 g (70.4% of theory) of the title product, m.p. 174 to 177°C.

Method G

To a solution of 4,9-dihydrothieno(2,3-c)-2-benzothiepin-4-ol (2.3 g) in anhydrous pyridine (30 ml) is added in one portion under stirring 1.9 g of p-toluenesulphochloride, and the mixture is stored for about 16 hours at room temperature. Thereafter, 1.4 g of imidazole is added and the whole is allowed to stand over for another 16-hour storage period at 20 to 25°C. After that the reaction mixture is diluted with about 50 ml of water, the product is shaken into chloroform, the organic layer is washed with water and dried over anhydrous sodium sulphate, and the solvent is removed under reduced pressure. The residue is mixed with about 5 ml of toluene, and the crystalline product is separated and washed with a small amount of toluene to give 0.8 g of the compound melting at 172 to 174°C.

Example 2

6-Chloro-4-(1-imidazolyl)-4,9-dihydrothieno(2,3-c)-2-benzothiepin

Method A

6-Chloro-4,9-dihydrothieno(2,3-c)-2-benzothiepin-4-ol (8.0 g) is dissolved by warming to about 50°C in 230 ml of dry benzene. The solution is cooled to 20°C and 1,1'-carbonyldiimidazole (9.7 g) is added thereto in one portion under stirring. The reaction mixture is stirred for 4 hours, then 50 ml of water is added and the organic phase is separated, washed with another 50 ml water portion, dried over anhydrous sodium sulphate, and the solvent is removed under reduced pressure. The residue is diluted with hexane (50 ml), and the formed crystalline precipitate of the product is separated and washed with 10 ml of the same diluent. The yield is 7.4 g (78% of theory) of the substance melting at 147 to 150°C. Further purification is possible by crystallization, eg from toluene.

Method B

A solution of imidazole (54.5 g) in anhydrous 1,2-dichloroethane (600 ml) is treated dropwise over a period of about 10 minutes under stirring and cooling to a temperature of 16 to 18°C with thionylchloride (23.8 g). The reaction mixture, in which imidazole hydrochloride precipitates, is stirred for 20 minutes at 18°C. During the next 5 minutes, 53.8 g of 6-chloro-4,9-dihydrothieno(2,3-c)-2-benzothiepin-4-ol is added portionwise while maintaining the temperature between 20 and 25°C by cooling with water. The reaction mixture is stirred within the same temperature range for 6 hours and after that it is allowed to stand for 16 hours. Thereafter, is is diluted with 500 ml of water and the separated organic phase is washed twice with 500 ml of water, dried over anhydrous sodium sulphate, filtered with active carbon, evaporated under reduced pressure to a volume of about 70 ml and allowed to stand for about 3 hours to crystallize. The crystals are collected on a filter and washed successively with toluene (20 ml) and hexane to give 45.6 g (71.5% of theory) of the pure product melting at 150 to 152°C. Analytically pure compound crystallizes from toluene and has the same melting temperature.

Nitrate salt of the title compound is obtained by acidification of a solution of the base, eg in chloroform with a mixture of 65% nitric acid and ethanol, and purified by crystallization from 2-propanol. The salt has a m.p. of 160 to 161°C.

The required starting material, 6-chloro-4,9-dihydrothieno(2,3-c)-2-benzothiepin-4-ol, which also is a novel compound, can be prepared by the following procedure.

6-Chlorothieno(2,3-c)-2-benzothiepin-4(9H)-one (53.3 g) is suspended in methanol (700 ml), and sodium borohydride (7.8 g) is added under stirring within approximately 1 hour. The reaction mixture is stirred for a further 3 hours and thereafter allowed to stand at 20 to 25°C. Crystals of the product are separated and washed successively with methanol (20 ml) and water (70 ml) to give 35.1 g of the first-crop product. The filtrate is evaporated to a volume of about 300 ml and diluted with water (500 ml) to afford second-crop crystals (17.1 g). The total yield of the intermediate is 52.2 g (97.1% of theory), m.p. 153 to 158°C. An analytical sample recrystallized from benzene - petroleum ether or from toluene has a m.p. of 156 to 158°C.

**Claims for the Contracting States: BE, CH, DE, FR, GB, IT, LI, LU, NL, SE**

1. Imidazolyl thieno-benzothiepin derivatives of the general formula I

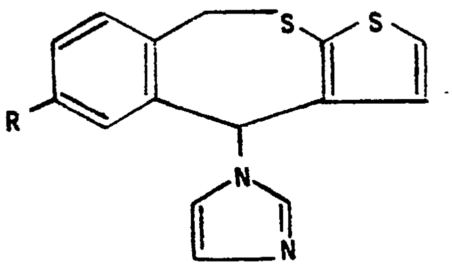

(I)

in which R is a hydrogen or a chlorine atom, and acid addition salts thereof with pharmaceutically acceptable inorganic and organic acids.

2. 4-(1-Imidazolyl)-4,9-dihydrothieno(2,3-c)-2-benzothiepin or an acid addition salt thereof.

3. 6-chloro-4-(1-Imidazolyl)-4,9-dihydrothieno(2,3-c)-2-benzothiepin or an acid addition salt thereof.

4. An acid addition salt according to any one of claims 1 to 3 wherein the acid used is nitric acid so as to obtain the nitrate.

5. 6-chloro-4,9-dihydrothieno-(2,3-c)-2-benzothiepin-4-ol.

6. An anti-mycotic agent in a topical or oral dosage form comprising as active ingredient a therapeutically effective amount of an imidazolyl thieno-benzothiepin derivative according to claim 1 with a pharmaceutically acceptable inorganic or organic acid, and where necessary to render said dosage administrable a pharmaceutically acceptable auxiliary.

7. An anti-mycotic agent according to claim 5 wherein the active ingredient is present in said agent in an amount of from 1 to 10% by weight.

8. An imidazolyl thieno-benzothiepin derivative according to claim 1 with a pharmaceutically acceptable organic or inorganic acid for use in the treatment of mycoses.

9. The use of an imidazolyl thieno-benzothiepin derivative according to claim 1 for the manufacture of a medicament for the treatment of mycoses.

10. A process for the preparation of imidazolyl thieno-benzothiepin derivatives according to claim 1, which comprises reacting the respective thieno-benzothiepin-4-ol compound of the general formula II

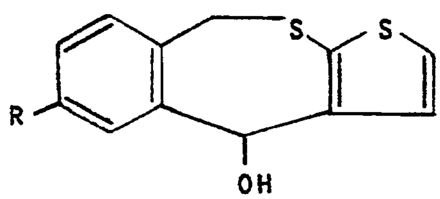

(II)

in which R is as defined in claim 1, or its alkane- or arenesulphonate ester with imidazole or a reactive derivative thereof, optionally with subsequent neutralisation of the resultant base with a pharmaceutically acceptable inorganic or organic acid.

**Claims for the Contracting State : AT**

1. A process for the preparation of imidazolyl thieno-benzothiepin derivatives of the general formula I

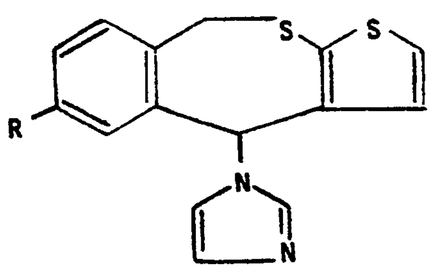

(I)

in which R is a hydrogen or a chlorine atom, and acid addition salts thereof, which comprises reacting the respective thieno-benzothiepin-4-ol compound of the general formula II

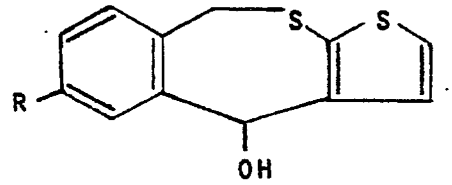

(II)

in which R has the above defined meaning, or its alkane- or arenesulphonate ester with imidazole or a reactive derivative thereof, optionally with subsequent neutralization of the resultant base with a pharmaceutically acceptable inorganic or organic acid.

2. A process according to claim 1 wherein said reaction of the said thieno-benzothiepin-4-ol compound is carried out in an inert organic solvent.

3. A process according to claim 1 or claim 2 wherein a thieno-benzothiepin-4-ol of formula II

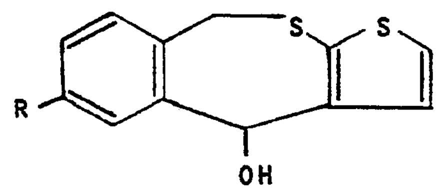

(II)

is reacted with 1,1′-carbonyldiimidazole in an anhydrous aromatic hydrocarbon, a chlorinated alkane, an aliphatic or cyclic ether or an aliphatic nitrile at a temperature substantially in the range of from 0 to 60°C.

4. A process of claim 1 wherein a thieno-benzothiepin-4-ol of formula II

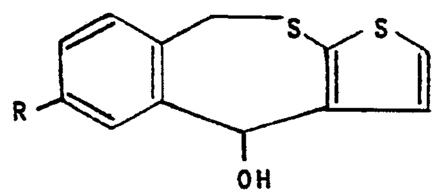

(II)

is reacted with 1,1′-thionyldiimidazole in an anhydrous aromatic hydrocarbon, a chlorinated alkane or an aliphatic nitrile at a temperature substantially in the range of from 10 to 25°C.

5. A process according to claim 4 wherein said 1,1′-thionyldiimidazole is prepared in situ by the reaction of imidazole with thionylchloride.

6. A process according to any one of claims 1 to 5 wherein the optional neutralisation step is carried out with nitric acid.

7. An imidazolyl thieno-benzothiepin derivative whenever prepared by a process substantially in accordance with any of the preceding claims.

8. A process for preparing 6-chloro-4,9-dihydrothieno-(2,3-c)-2-benzothiepin-4-ol which comprises reduction of 6-chlorothieno(2,3-c)-2-benzothiepin-4(9H)-one with a hydride reducing agent in a suitable alcoholic solvent.

9. A process according to claim 8 wherein the reducing agent is sodium borohydride.

10. A process according to claim 8 or claim 9 wherein the starting ketone is suspended in methanol.

**Patentansprüche für die Vertragsstaaten: BE, CH, DE, FR, GB, IT, LI, LU, NL, SE**

1. Imidazolylthieno-benzothiepinderivate der allgemeinen Formel I

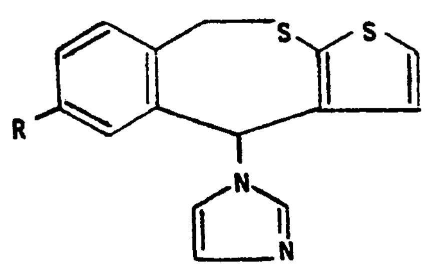

(I)

in welcher R Wasserstoff oder ein Chloratom bedeutet und Säureadditionssalze davon mit pharmazeutisch verträglichen anorganischen und organischen Säuren.

2. 4-(1-Imidazolyl)-4,9-dihydrothieno-(2,3-c)-2-benothiepin oder ein Säureadditionssalz davon.

3. 6-Chlor-4-(1-imidazolyl)-4,9-dihydrothieno-(2,3-c)-2-benzothiepin oder ein Säureadditionssalz davon.

4. Säureadditionssalz nach einem der Ansprüche 1 bis 3, worin die verwendete Säure Salpetersäure ist, um das Nitrat zu erhalten.

5. 6-Chlor-4,9-dihydrothieno-(2,3-c)-2-benzothiepin-4-ol.

6. Antimykotikum in einer für topische oder orale Anwendung geeigneten Dosierungsform, welche als Wirksubstanz eine therapeutisch wirksame Menge eines Imidazolylthieno-benzothiepinderivates nach Anspruch 1 mit einer pharmazeutisch verträglichen anorganischen oder organischen Säure und gegebenenfalls, um diese Dosis verabreichbar zu machen, einen pharmazeutisch verträglichen Hilfsstoff enthält.

7. Antimykotikum nach Anspruch 5, worin der Wirkstoff in dem Agens in einer Menge von 1 bis 10 Gew.-% vorhanden ist.

8. Imidazolylthieno-benzothiepinderivat nach Anspruch 1 mit einer pharmazeutisch verträglichen organischen oder anorganischen Säure, zur Verwendung bei der Behandlung von Mykosen.

9. Verwendung eines Imidazolylthieno-benzothiepinderivates nach Anspruch 1 zur Herstellung eines Pharmazeutikums zur Behandlung von Mykosen.

10. Verfahren zur Herstellung von Imidazolylthieno-benzothiepinderivaten nach Anspruch 1, welches die Umsetzung der entsprechenden Thienobenzothiepin-4-ol-Verbindung der allgemeinen Formel II

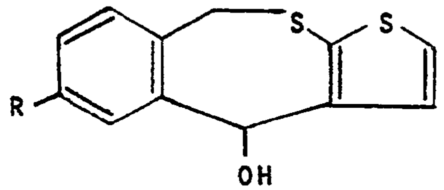

(II)

in welcher R wie in Anspruch 1 definiert ist, oder ihre Alkan- oder Arensulfonatester mit Imidazol oder einem reaktiven Derivat davon umfaßt, gegebenenfalls mit nachfolgender Neutralisation der resultierenden Base mit einer pharmazeutisch verträglichen anorganischen oder organischen Säure.

**Patentansprüche für den Vertragsstaat: AT**

1. Verfahren zur Herstellung von Imidazolylthieno-benzothiepinderivaten der allgemeinen Formel I

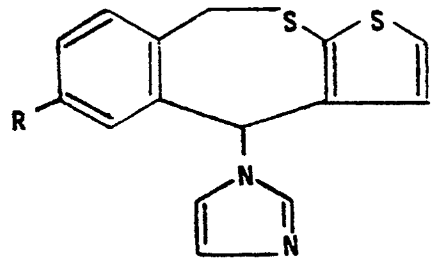

(I)

in welcher R Wasserstoff oder ein Chloratom bedeutet und Säureadditionssalze davon, welches die Umsetzung der entsprechenden Thienobenzothiepin-4-ol-Verbindung der allgemeinen Formel II

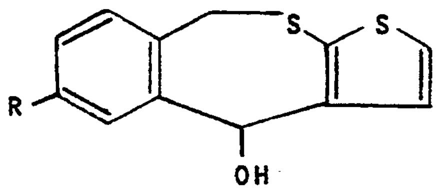

(II)

in welcher R die oben angegebene Bedeutung besitzt, oder ihrer Alkan- oder Arensulfonatester mit Imidazol oder einem reaktiven Derivat davon umfaßt, gegebenenfalls mit nachfolgender Neutralisation der resultierenden Base mit einer pharmazeutisch verträglichen anorganischen oder organischen Säure.

2. Verfahren nach Anspruch 1, worin die Reaktion der Thienobenzothiepinverbindung in einem inerten organischen Lösungsmittel durchgeführt wird.

3. Verfahren nach Anspruch 1 oder 2, worin ein Thienobenzothiepin-4-ol der Formel II

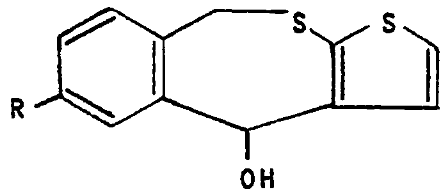

(II)

mit 1,1'-Carbonyldiimidazol in einem wasserfreien aromatischen Kohlenwasserstoff, einem chlorierten Alkan, einem aliphatischen oder cyclischen Ether oder einem aliphatischen Nitril bei einer Temperatur im Bereich von 0 bis 60°C durchgeführt wird.

4. Verfahren nach Anspruch 1, worin ein Thienobenzothiepin-4-ol der Formel II

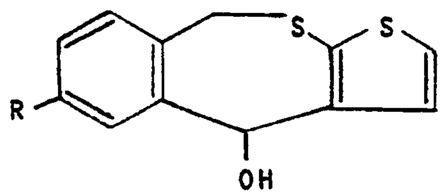

(II)

mit 1,1'-Thionyldiimidazol in einem wasserfreien aromatischen Kohlenwasserstoff, einem chlorierten Alkan oder einem aliphatischen Nitril bei einer Temperatur im Bereich von 10 bis 25°C umgesetzt wird.

5. Verfahren nach Anspruch 4, worin das 1,1'-Thionyldiimidazol in situ durch Reaktion von Imidazol mit dem Thionylchlorid hergestellt wird.

6. Verfahren nach einem der Ansprüche 1 bis 5, worin der gegebenenfalls vorhandene Neutralisationsschritt in Salpetersäure durchgeführt wird.

7. Imidazolylthieno-benzothiepinderivat, welches nach dem Verfahren von einem der vorhergehenden Ansprüche hergestellt wird.

8. Verfahren zur Herstellung von 6-Chlor-4,9-dihydrothieno-(2,3-c)-2-benzothiepin-4-ol, welches die Reaktion von 6-Chlorthieno-(2,3-c)-2-benzothiepin-4(9H)-on mit einem Hydridreduktionsmittel in einem geeigneten alkoholischen Lösungsmittel umfaßt.

9. Verfahren nach Anspruch 8, worin das Reduktionsmittel Natriumborhydrid ist.

10. Verfahren nach Anspruch 8 oder 9, worin das Ausgangsketon in Methanol suspendiert ist.

**Revendications pour les Etats contractants: BE, CH, DE, FR, GB, IT, LI, LU, NL, SE**

1. Dérivés d'imidazole-thiéno-benzothiépine de formule générale I:

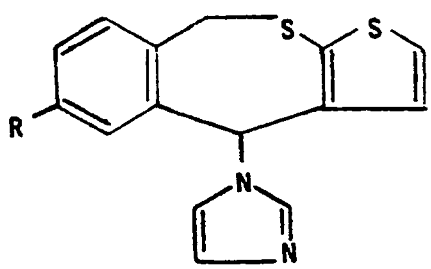

(I)

dans laquelle R est un atome d'hydrogène ou de chlore, et sels d'addition d'acide de ceux-ci avec des acides organiques ou minéraux pharmaceutiquement acceptables.

2. 4-(1-imidazol)-4,9-dihydrothiéno-(2,3-c)-2-benzothiépine ou sel d'addition d'acide de celle-ci.

3. 6-chloro-4-(1-imidazolyl)-4,9-dihydrothiéno-(2,3-c)-2-benzothiépine ou sel d'addition d'acide de celle-ci.

4. Sel d'addition d'acide selon l'une quelconque des revendications 1 à 3, dans lequel l'acide utilisé est l'acide nitrique, de manière à obtenir le nitrate.

5. 6-chloro-4,9-dihydrothiéno-(2,3-c)-2-benzothiépine-4-ol.

6. Agent antimycotique sous une forme pharmaceutique ovale ou locale, comprenant en tant que composant actif une quantité thérapeutiquement efficace d'un dérivé d'imidazolyl-thiéno-benzothiépine selon la revendication 1, avec un acide organique ou minéral pharmaceutiquement acceptable, et, lorsque cela est nécessaire pour rendre ladite forme administrable, une substance auxiliaire pharmaceutiquement acceptable.

7. Agent antimycotique selon la revendication 5, dans lequel le composant actif est présent dans ledit agent en une quantité de 1 à 10% en poids.

8. Dérivé d'imidazolyl-thiéno-benzothiépine selon la revendication 1, avec un acide organique ou minéral pharmaceutiquement acceptable, pour utilisation dans le traitement de mycoses.

9. Utilisation d'un dérivé d'imidazolyl-thiéno-benzothiépine selon la revendication 1, pour la fabrication d'un médicament pour le traitement de mycoses.

10. Procédé pour la préparation de dérives d'imidazolyl-thiéno-benzothiépine selon la revendication 1, lequel comprend la mise en réaction du composé thiéno-benzothiépine-4-ol respectif, de formule générale II:

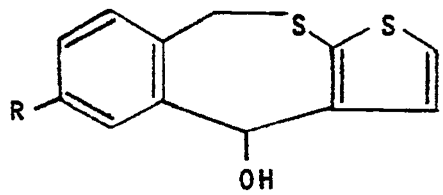

(II)

dans laquelle R est tel que défini dans la revendication 1, ou d'un ester alcane- ou arènesulfonique de celui-ci, avec l'imidazole ou un dérivé réactif de celui-ci, éventuellement avec neutralisation subséquente de la base résultante par un sel organique ou minéral pharmaceutiquement acceptable.

**Revendications pour l'Etat contractant: AT**

1. Procédé pour la préparation de dérivés d'imidazolyl-thiéno-benzothiépine de formule générale I:

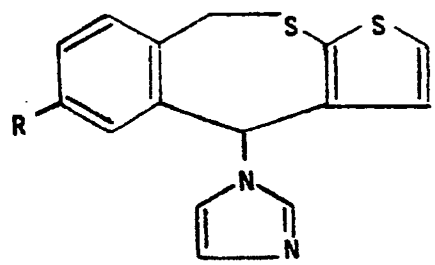

(I)

dans laquelle R est un atome d'hydrogène ou de chlore, et de leurs sels d'addition avec des acides, lequel comprend la mise en réaction du composé thiéno-bnezothiépine-4-ol respectif, de formule générale II:

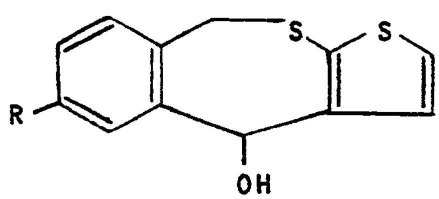

(II)

dans laquelle R a la signification donnée ci-dessus, ou d'un ester alcane- ou arènesulfonique de celui-ci, avec l'imidazole ou un dérivé réactif de celui-ci, éventuellement avec neutralisation subséquente de la base résultante par un acide organique ou minéral pharmaceutiquement acceptable.

2. Procédé selon la revendication 1, dans lequel ladite réaction dudit composé thiéno-benzothiépine-4-ol est effectuée dans un solvant organique inerte.

3. Procédé selon la revendication 1 ou 2, dans lequel on fait réagir un thiéno-benzothiépine-4-ol de formule II:

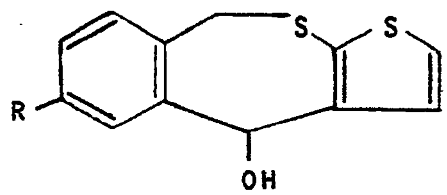

(II)

avec le 1,1'-carbonyldiimidazole dans un hydrocarbure aromatique anhydre, un alcane chloré, un éther aliphatique ou cyclique ou un nitrile aliphatique, à une température essentiellement dans la plage de 0 à 60°C.

4. Procédé selon la revendication 1, dans lequel on fait réagir un thiéno-benzothiépine-4-ol de formule II:

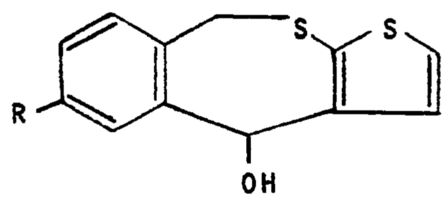

(II)

avec le 1,1-thionyldiimidazole dans un hydrocarbure aromatique anhydre, un alcane chloré ou un nitrile aliphatique, à une température essentiellement dans la plage de 10 à 25°C.

5. Procédé selon la revendication 4, dans lequel ledit 1,1'-thionyldiimidazole est préparé in situ par la réaction de l'imidazole avec le chlorure de thionyle.

6. Procédé selon l'une quelconque des revendications 1 à 5, dans lequel l'étape éventuellement de neutralisation est effectuée avec de l'acide nitrique.

7. Dérivé d'imidazolyl-thiéno-benzothiépine, lorsqu'il est préparé par un procédé essentiellement conforme à l'une quelconque des revendications précédentes.

8. Procédé pour la préparation de 6-chloro-4,9-dihydrothiéno-(2,3-c)-2-benzothiépine-4-ol, lequel comprend la réduction de la 6-chlorothiéno-(2,3-c)-2-benzothiépine-4(9H)-one par un réducteur de type hydrure dans un solvant alcoolique approprié.

9. Procédé selon la revendication 8, dans lequel le réducteur est le borohydrure de sodium.

10. Procédé selon la revendication 8 ou 9, dans lequel la cétone de départ est en suspension dans du méthanol.